Europäisches Patentamt
European Patent Office
Office européen des brevets

(19)

(11) Veröffentlichungsnummer: **0 347 377 A2**

(12)

# EUROPÄISCHE PATENTANMELDUNG

(21) Anmeldenummer: **89810422.9**

(22) Anmeldetag: **05.06.89**

(51) Int. Cl.⁴: **C 07 D 285/14**
A 01 N 43/82, C 07 C 149/43,
C 07 C 149/40,
C 07 D 417/12, C 07 F 7/08,
C 07 F 9/40, C 07 H 13/08

(30) Priorität: **13.06.88 CH 2256/88**

(43) Veröffentlichungstag der Anmeldung:
**20.12.89 Patentblatt 89/51**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Schurter, Rolf, Dr.**
**Holzmattstrasse 45**
**CH-4102 Binningen (CH)**

(54) **1,2,3-Benzthiadiazol-4-carbonsäure-Derivate als Pflanzenwuchsregulatoren.**

(57) Die Erfindung betrifft neue 1,2,3-Benzthiadiazol-4-carbonsäure-Derivate mit pflanzenregulatorischen insbesondere den Pflanzenwuchs hemmenden Eigenschaften, welche der Formel I entsprechen,

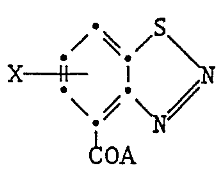

(I)

in welcher
X Wasserstoff, Halogen, $C_1$-$C_3$-Alkyl, $C_1$-$C_3$-Alkoxy oder $C_1$-$C_3$-Alkylthio,
A einen Rest -OR, -SR oder $-ON=C(R_1)R_2$,
R Wasserstoff, ein Metall- oder Ammoniumkation, einen unsubstituierten oder substituierten Alkyl-, Alkenyl-, Alkinyl-, Cycloalkyl- oder Arylrest, einen heterocyclischen Rest oder einen Monosaccharid-Rest,
$R_1$ und $R_2$ unabhängig voneinander je Wasserstoff, $C_1$-$C_6$-Alkyl, $C_2$-$C_8$-Alkoxyalkyl, Cyano, unsubstituiertes oder substituiertes Phenyl oder einen substituierten oder unsubstituierten Alkanoyl- oder Alkenoyl-Rest,
$R_1$ und $R_2$ zusammen mit dem Kohlenstoffatom, an das sie gebunden sind, bilden auch einen heterocyclischen oder einen carbocyclischen Rest mit 3-7 Ringatomen bedeuten.

Die Herstellung dieser Derivate sowie von einigen neuen Zwischenprodukten, ausgehend von 3-Halo-2-nitro-benzoesäuren und Ester werden beschrieben.

EP 0 347 377 A2

**Beschreibung**

## 1,2,3-Benzthiadiazol-4-carbonsäure-Derivate als Pflanzenwuchsregulatoren

Die vorliegende Erfindung betrifft neue 1,2,3-Benzthiadiazol-4-carbonsäure-Derivate sowie ein Mittel zur Regulierung des Pflanzenwuchses, welches als Wirkstoffe substituierte 1,2,3-Benzthiadiazol-4-carbonsäure-Derivate der nachstehenden Formel enthalten. Die Erfindung betrifft ferner die Herstellung der genannten Mittel sowie die Herstellung der Wirkstoffe. Die Erfindung betrifft darüber hinaus die Verwendung der Wirkstoffe oder der Mittel zum Regulieren, insbesondere zum Hemmen des Pflanzenwuchses.

Die 1,2,3-Benzthiadiazol-4-carbonsäure-Derivate entsprechen der allgemeinen Formel I

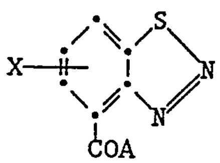

(I)

in welcher
X Wasserstoff, Halogen, $C_1$-$C_3$-Alkyl, $C_1$-$C_3$-Alkoxy oder $C_1$-$C_3$-Alkylthio,
A einen Rest -OR, -SR oder $-ON=C(R_1)R_2$,
R Wasserstoff, ein Metall- oder Ammoniumkation, einen unsubstituierten oder substituierten Alkyl-, Alkenyl-, Alkinyl-, Cycloalkyl- oder Arylrest, einen heterocyclischen Rest oder einen Monosaccharid-Rest, $R_1$ und $R_2$ unabhängig voneinander je Wasserstoff, $C_1$-$C_6$-Alkyl, $C_2$-$C_8$-Alkoxyalkyl, Cyan, einen unsubstituierten oder substituierten Phenyl-, oder einen substituierten oder unsubstituierten Alkanoyl- oder Alkenoyl-Rest bedeuten und
$R_1$ und $R_2$ zusammen mit dem Kohlenstoffatom an das sie gebunden sind, auch einen heterocyclischen oder einen carbocyclischen Rest mit 3-7 Ringatomen bilden können.

In dieser Definition bedeuten R, $R_1$ und $R_2$ speziell:
R Wasserstoff, ein Alkalimetall-, Erdalkalimetall, Aluminium-, Kupfer-, Nickel-, Mangan-, Zink-, Eisen- oder Silberkation, den Rest einer Base oder ein Ammoniumkation der Formel

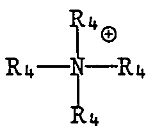 ,

wobei die $R_4$ unabhängig voneinander durch Wasserstoff, $C_1$-$C_6$-Alkyl, $C_1$-$C_6$-Hydroxyalkyl, $C_2$-$C_6$-Alkoxyalkyl, $C_1$-$C_6$-Halogenalkyl, $C_3$-$C_6$-Alkenyl, Benzyl, Pyridinyl vertreten sein können, zwei $R_4$ können zusammen auch eine $C_3$-$C_7$-Alkylenkette bilden, die durch Sauerstoff unterbrochen sein kann,
R ist ferner $C_1$-$C_8$-Alkyl, durch Halogen, Cyano, Nitro, Hydroxy oder U-$C_1$-$C_3$-Alkyl substituiertes $C_1$-$C_8$-Alkyl, (T)-CO-$C_1$-$C_4$-Alkyl, (T)-COOH oder (T)-COO$C_1$-$C_4$-Alkyl, $C_2$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl oder eine Gruppe aus der Reihe

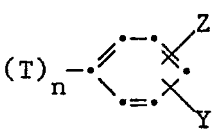 , $(T)_n$-Naphthyl(Y), $(T)_n$-Si($C_1$-$C_8$-Alkyl$)_3$, (T)-$NR_2R_3$,

$-N=C(R_1)R_2$, 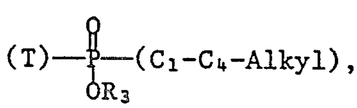 , 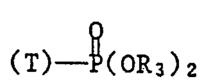 oder $(T)_n$—W ;

W einen 5- bis 7-gliedrigen gesättigten oder ungesättigten unsubstituierten oder durch Halogen, Trifluormethyl, Cyano oder $C_1$-$C_2$-Alkyl substituierten Heterocyclus mit 1 bis 3 Heteroatomen aus der Gruppe O, N und S sowie darüber hinaus einen Monosaccharid-Rest,
T unverzweigtes oder verzweigtes $C_1$-$C_8$-Alkylen, besonders-$CH_2$-, -$CH_2CH_2$-, -$CH_2$-$CH_2$-$CH_2$-, -$CH(CH_3)$- oder -$CCH_3(CH_3)$- sowie $C_3$-$C_6$-Alkenylen oder $C_3$-$C_6$-Alkinylen,
T kann durch Sauerstoff oder CO unterbrochen sein und gegebenenfalls durch Halogen, Hydroxy, $C_1$-$C_5$-Alkoxy, $C_3$-$C_6$-Cycloalkyl, $C_1$-$C_5$-Alkoxycarbonyl, $C_1$-$C_5$-Alkyl, $C_1$-$C_2$-Haloalkyl, CN oder COOH substituiert sein, T kann auch Teil eines Cycloalkylrestes sein,
n Null oder 1,
$R_1$ und $R_2$ unabhängig voneinander je Wasserstoff, $C_1$-$C_6$-Alkyl, $C_2$-$C_8$-Alkoxyalkyl, Cyan, einen unsubstituierten oder substituierten Phenylrest, $C_1$-$C_3$-Alkanoyl, durch Halogen oder $C_1$-$C_3$-Alkoxy substituiertes

$C_1$-$C_3$-Alkanoyl, $C_3$-$C_5$-Alkenoyl oder durch Halogen oder $C_1$-$C_3$-Alkoxy substituiertes $C_3$-$C_5$-Alkenoyl,
$R_1$ und $R_2$ zusammen mit dem Kohlenstoffatom, an das sie gebunden sind, einen Heterocyclus W oder einen carbocyclischen Rest mit 3- bis 7 Ringkohlenstoffatomen,
Y und Z Wasserstoff, Halogen, $C_1$-$C_4$-Alkyl, $C_1$-$C_4$-Haloalkyl, $C_1$-$C_4$-Alkoxy, $C_1$-$C_4$-Haloalkoxy, Cyan, $NO_2$, $C_1$-$C_4$-Alkoxycarbonyl oder $C_1$-$C_4$-Alkylcarbonyl,
$R_3$ Wasserstoff oder $C_1$-$C_4$-Alkyl und
U Sauerstoff oder Schwefel.

Von den erfindungsgemässen Verbindungen sind im Falle von A = OR die Salze der Carbonsäure mit primären, sekundären und tertiären Aminen, Metall- und Ammoniumkationen umfasst. Halogen selbst oder als Bestandteil eines anderen Substituenten bedeutet Fluor, Chlor, Brom oder Jod, vorzugsweise Fluor, Chlor oder Brom. Unter Alkyl selbst oder als Bestandteil eines anderen Substituenten sind gerad- oder verzweigtkettige Alkyle zu verstehen. Sie stellen je nach Zahl der angegebenen Kohlenstoffatome beispielsweise folgende Gruppen dar: Methyl, Ethyl sowie die Isomeren von Propyl, Butyl, Pentyl, Hexyl, Heptyl oder Octyl wie z.B. Isopropyl, Isobutyl, tert.-Butyl, sek.-Butyl oder Isopentyl. Cycloalkyl bedeutet z.B. Cyclopropyl, Cyclobutyl, Cyclopentyl oder Cyclohexyl.

Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw., vorzugsweise Propargyl.

Als Basen zur Bildung der Salze kommen anorganische oder organische in Betracht, wie z.B. Hydroxide, Carbonate und Hydrogencarbonate der Alkali- und Erdalkalimetalle, vorzugsweise LiOH, NaOH, KOH, Mg(OH)z oder $Ca(OH)_2$ und ferner $NaHCO_3$, $KHCO_3$, $Na_2CO_3$ und $K_2CO_3$.

Als salzbildende Amine sind z.B. anzusehen:
Trimethylamin, Triethylamin, Tripropylamin, Tributylamin, Tribenzylamin, Tricyclohexylamin, Triamylamin, Trihexylamin, N,N-Dimethylanilin, N,N-Dimethyltoluidin, N,N-Dimethyl-p-amino-pyridin, N-Methylpyrrolidin, N-Methylpiperidin, N-Methyl-pyrrolidin, N-Methylimidazol, N-Methylpyrrol, N-Methylmorpholin, N-Methylhexamethylenimin, Pyridin, Chinolin, alpha-Picolin, β-Picolin, Isochinolin, Pyrimidin, Acridin, N,N,N',N'-Tetra-methylethylendiamin, N,N,N',N'-Tetraethylethylendiamin, Chinoxalin, N-Propyldiisopropylamin, N,N-Dimethylcyclohexylamin, 2,6-Lutidin, 2,4-Lutidin, Triethylendiamin sowie heterocyclische Amine vom Morpholintyp.

Unter Heterocyclen sind zu verstehen z.B.: Furan, Tetrahydrofuran, Thiophen, Tetrahydropyran, Pyrrol, Pyrrolidin, Imidazol, 1,2,4-Triazol, Piperidin, Pyridin, Pyrimidin, Morpholin oder Azacycloheptan. Insbesondere stellen sie dar: Furan-2-yl, Tetrahydrofuran-2-yl, Tetrahydrofuran-3-yl, Tetrahydropyran-2-yl, Pyrrol-1-yl, Pyrrol-2-yl, Pyrrolidin-1-yl, Imidazol-1-yl, 1,2,4-Triazol-1-yl, 1,3,4-Triazol-1-yl, Thiophen-2-yl, Piperidin-1-yl, Piperidin-4-yl, Pyridin-2-yl, Pyridin-3-yl, Pyridin-4-yl, Pyrimidin-2-yl, Pyrimidin-4-yl, Pyrimidin-5-yl, Morpholin-1-yl oder Azacycloheptan-1-yl.

Unter einem Monosaccharid-Rest sind beispielsweise zu verstehen Glucofuranosyl, Galaktopyranosyl, Allofuranosyl oder Mannityl, wobei die OH-Gruppen frei oder acetyliert oder mit Methyl, Benzyl oder Isopropylidenyl verethert sind. Unter den genannten Resten sind die Diisopropylidenyl-Derivate bevorzugt, während von diesen wiederum besonders bevorzugte Reste sind: Diaceton-D-glucosidyl, 1,2,3,4-Di-O-isopropyliden-D-galactopyranos-6-yl, 1,2,5,6-Di-O-isopropyliden-D-mannit-3-yl, 1,2,5,6-Di-O-isopropyliden-α-D-allofuranos-3-yl, D-Glucofuranos-3-yl, D-Galactopyranos-6-yl, D-Mannit-3-yl, D-Allofuranos-4-yl, Mannopyranos-1-yl, 2-Methyl-D-glucosid-6-yl, 1,2,5,6-Tetraacetyl-D-galactopyranos-3-yl und 2,3,5-Tribenzylribofuranos-1-yl.

Aufgrund ihrer ausgeprägten den Pflanzenwuchs regulierenden und insbesondere hemmenden Eigenschaften sind diejenigen Wirksubstanzen bevorzugt, deren Strukturen folgende Substituenten oder Kombinationen dieser Substituenten untereinander aufweisen:

Die 1,2,3-Benzthiadiazol-4-carbonsäure-Derivate der Formel I,

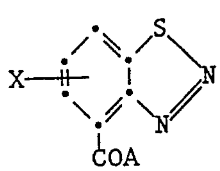

(I)

worin
X Wasserstoff, Halogen, $C_1$-$C_3$-Alkyl, $C_1$-$C_3$-Alkoxy oder $C_1$-$C_3$-Alkylthio, worin A einen Rest -OR, -SR oder $-ON=C(R_1)R_2$,
R Wasserstoff, ein Alkalimetallkation, $C_1$-$C_8$-Alkyl, $C_3$-$C_4$-Alkenyl, $C_3$-$C_4$-Alkinyl, $C_3$-$C_6$-Cyclohexyl, Phenyl oder Benzyl wobei der Phenylkern durch $C_1$-$C_3$-Alkyl, $C_1$-$C_3$-Haloalkyl, $C_1$-$C_3$-Alkoxy, $C_1$-$C_3$-Alkylthio, Cyan, Halogen, Nitro oder Methoxycarbonyl substituiert sein kann,
$R_1$ und $R_2$ unabhängig voneinander je $C_1$-$C_6$-Alkyl, $C_2$-$C_8$-Alkoxyalkyl oder $R_1$ und $R_2$ zusammen eine 2-6 gliedrige Alkylen- oder Alkenylenkette, bedeuten.

Die 1,2,3-Benzthiadiazol-4-carbonsäurederivate der Formel Ia

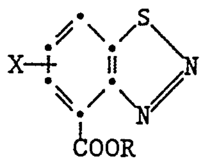

(Ia)

worin X und R die oben gegebene Bedeutung haben, insbesondere die
1,2,3-Benzthiadiazol-4-carbonsäuremethylester,
5-Chlor-1,2,3-benzthiadiazol-4-carbonsäuremethylester,
5-Chlor-1,2,3-benzthiadiazol-4-carbonsäure,
5-Chlor-1,2,3-benzthiadiazol-4-carbonsäureethylester,
5-Chlor-1,2,3-benzthiadiazol-4-carbonsäurebenzylester,
5-Methoxy-1,2,3-benzthiadiazol-4-carbonsäure-methylester,
5-Methylthio-1,2,3-benzthiadiazol-4-carbonsäure-methylester,
5-Chlor-1,2,3-benzthiadiazol-4-carbonsäure-n-butylester,
5-Chlor-1,2,3-benzthiadiazol-4-carbonsäure-phenylester.
5-Chlor-1,2,3-benzthiadiazol-4-carbonsäure-(2-chlorphenyl)-ester,
5-Chlor-1,2,3-benzthiadiazol-4-carbonsäure-propinylester,
5-Chlor-1,2,3-benzthiadiazol-4-carbonsäure-(trimethylsilylethyl)-ester,
5-Chlor-1,2,3-benzthiadiazol-4-carbonsäure-( dimethoxyphosphonylethyl)-ester und das Kaliumsalz der 5-Chlor-4-(1,3-dioxan-2-yl)-1,2,3-benzthiazol-4-carbonsäure.

Die 1,2,3-Benzthiadiazol-4-carbonsäurethiolester der Formel Ib

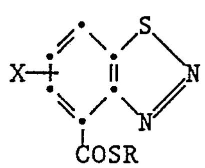

(Ib)

worin X und R die oben gegebene Bedeutung haben, insbesondere die
1,2,3-Benzthiadiazol-4-carbonsäure-benzylthiolester,
5-Chlor-1,2,3-benzthiadiazol-4-carbonsäure-methylthiolester,
5-Methylthio-1,2,3-benzthiadiazol-4-carbonsäuremethylthiolester, und
5-Chlor-1,2,3-benzthiadiazol-4-carbonsäure-(methoxycarbonylmethylthiol)-ester.

Die 1,2,3-Benzthiadiazol-4-carbonsäure-oximester der Formel Ic

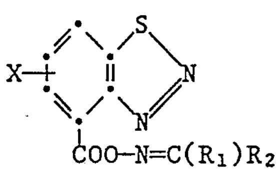

(Ic)

worin $R_1$, $R_2$ und x die oben gegebene Bedeutung haben, insbesondere der 5-Chlor-1,2,3-benzthiadiazol-4-carbonsäure-acetoxim-ester.

Die unter die Formel I fallenden 1,2,3-Benzthiadiazol-4-carbonsäurederivate sind neu. In der Deutschen Offenlegungsschrift Nr. 1 695 786 und in der französischen Patentschrift Nrw. 1 541 415 sind 1,2,3-Benzthiadiazole in allgemeiner Form als biozide Wirkstoffe zur Anwendung in herbiziden, insektiziden und fungiziden Mitteln offenbart. Keine dieser Verbindungen fällt unter die Formel I der vorliegenden Erfindung, noch ist für sie eine pflanzenregulierende Aktivität beschrieben. Darüber hinaus ist die Benzo-1,2,3-thiadiazol-7-carbonsäure ohne Angabe biologischer Eigenschaften aus J. Chem. Soc. (C) 1971, 3997 bekannt.

Die Herstellung der 1,2,3-Benzthiadiazol-4-carbonsäurederivate erfolgt nach folgendem, an sich bekannten Verfahren

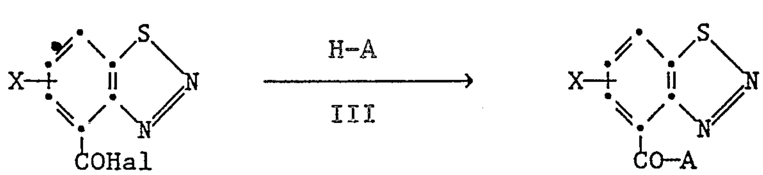


II I

Ein 1,2,3-Benzthiadiazol-4-carbonsäurehalogenid der Formel II wird in einem inerten organischen Lösungsmittel in Gegenwart einer Base mit einem Alkohol, Thiol oder Oxim der Formel HA umsetzt. Die

Umsetzung der Reaktanden HA = R-OH, HA = R-SH oder HA = HON=C($R_1$)$R_2$ geschieht mit oder ohne Zusatz eines 4-Alkylaminopyridins als Katalysator.

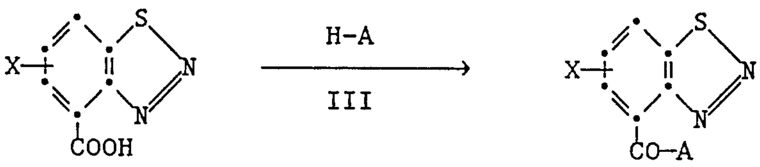


If I

Eine 1,2,3-Benzthiadiazol-4-carbonsäure der Formel If wird in einem wasserfreien organischen Lösungsmittel mit einem Alkohol, Thiol oder Oxim H-A der Formel III, in Gegenwart eines wasserentziehenden Mittels oder wasserentziehenden Bedingungen verestert oder kondensiert. Bei dieser Umsetzung wird H-A im Ueberschuss, in einem inerten Lösungsmittel, in Gegenwart einer Säure, wie Schwefelsäure, Salzsäure, p-Toluolsulfonsäure oder Bortrifluorid-Diethylether-Komplex, oder von Dicyclohexylcarbodiimid bei Temperaturen von -10° bis 180°C, vorzugsweise 0° bis 140°C, eingesetzt.

Die als Vorstufen benötigten 1,2,3-Benzthiadiazolcarbonsäureester der Formel Ia, worin R substituiertes oder unsubstituiertes $C_1$-$C_6$-Alkyl bedeutet, die Carbonsäuren der Formel If und die Säurehalide der Formel II werden nach an sich bekannten Methoden, z.B. entsprechend dem unten skizzierten Syntheseweg hergestellt.

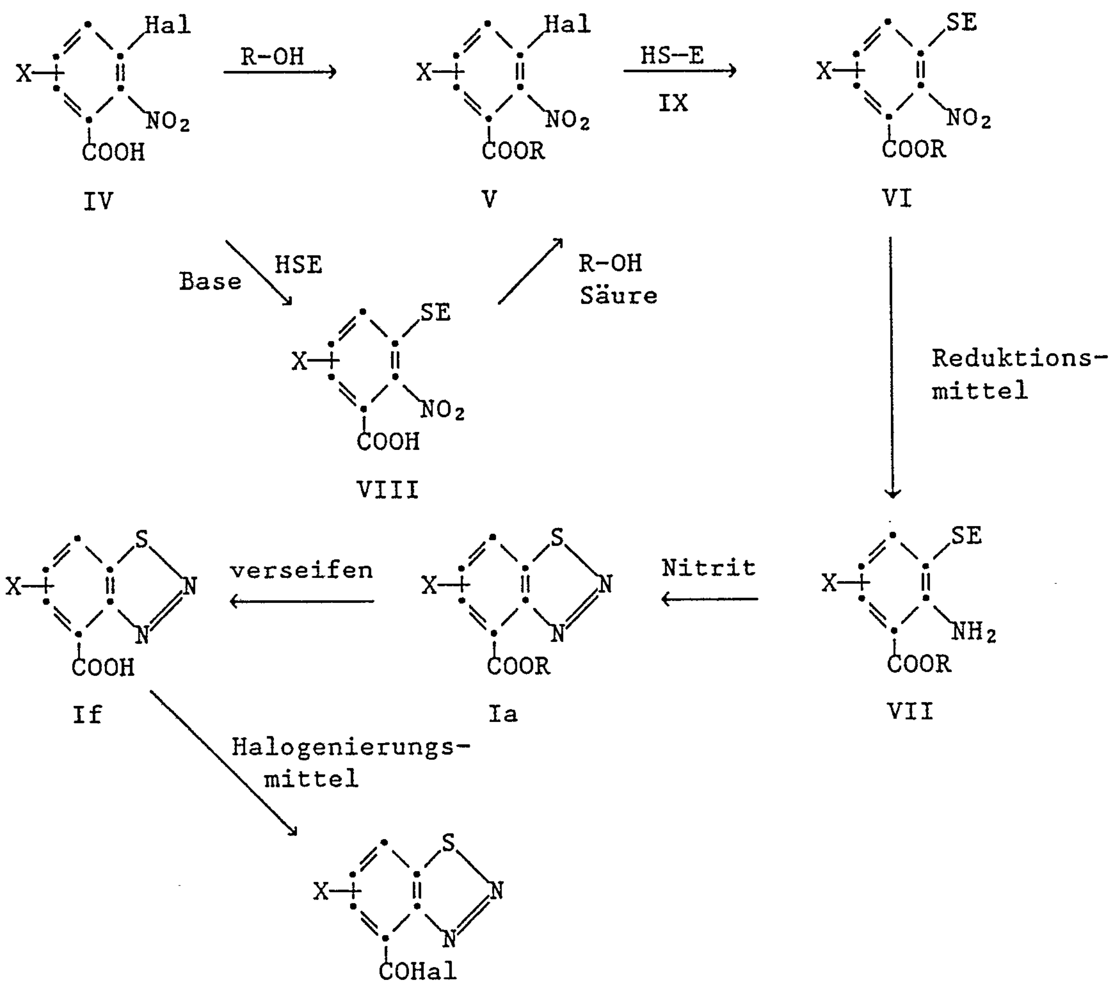


In diesen Formeln bedeuten E eine leicht abspaltbare Gruppe wie z.B. Wasserstoff, ein unsubstituierter oder substituierter $C_1$-$C_{12}$-Alkylrest, wie z.B. Methyl, Ethyl, Isopropyl, Dodecyl oder auch Benzyl. Hal steht für ein Halogenatom, vorzugsweise Chlor oder Brom während R und X die unter der Formel I gegebenen Bedeutungen haben. In diesen Umsetzungen ist R vorzugsweise Wasserstoff oder gegebenenfalls mit Phenyl

substituiertes $C_1$-$C_6$-Alkyl.

Die 3-Halo-2-nitrobenzoesäure der Formel IV wird in konventioneller Weise mit einem Ueberschuss eines Alkanols R-OH, z.B. in Gegenwart von etwas konzentrierter Schwefelsäure verestert, der Ester der Formel V anschliessend in einem inerten organischen Lösungsmittel in Gegenwart der äquimolaren Menge einer Base mit einem Mercaptan HS-E kondensiert. Der entstandene 3-Mercapto-2-nitrobenzoesäureester der Formel VI wird anschliessend mit einem Reduktionsmittel zum 2-Amino-3-mercaptobenzoesäureester der Formel VII reduziert. Durch Behandeln mit einem Nitrit, z.B. Natriumnitrit, findet Ringschluss zum 1,2,3-Benzthiadiazol-4-carbonsäureester entsprechend der Formel Ia statt, welcher dann auf konventionelle Art und Weise zur 1,2,3-Benzthiadiazol-4-carbonsäure der Formel If verseift und diese weiter mit einem Halogenierungsmittel wie Thionylchlorid, Phosphoroxychlorid oder den entsprechenden Bromiden zum 1,2,3-Benzthiadiazol-4-carbonsäurehalid der Formel II umgewandelt wird.

Die Cyclisierung der Verbindung der Formel VII in welcher E eine leicht abspaltbare Gruppe, wie z.B. Wasserstoff, $C_1$-$C_{12}$-Alkyl, z.B. Methyl, Ethyl oder die Benzylgruppe darstellt, wird in saurem Medium mit einer Nitritverbindung bei -20° bis 30°C diazotiert. Dabei finden als saures Reaktionsmedium wässerige Verdünnungen von anorganischen Säuren, wie z.B. Halogenwasserstoffsäuren, Phosphorsäure, Schwefelsäure oder Borfluorwasserstoffsäure, Anwendung; jedoch können auch geeignete organische Säuren verwendet werden. Als Nitrite sind sowohl anorganische Nitrit-Verbindungen, wie z.B. die Alkali- und Erdalkalisalze, wie auch organische Nitrit-Verbindungen, wie z.B. Alkylnitrite, geeignet.

Diese Methode des Ringschlusses, wobei E ein unsubstituierter oder substituierter $C_1$-$C_{12}$-Alkylrest ist, stellt ein Verfahren dar, mit welchem annellierte Thiadiazolverbindungen in vorteilhafter Weise zugänglich sind (s. A. Buraway et al., J. Chem. Soc. 90, p. 1354).

Die Umwandlung der Verbindung der Formel V zur Verbindung der Formel VI geschieht in Gegenwart von einem Aequivalent einer Base, wie beispielsweise einem Alkalicarbonat (z.B. $Na_2CO_3$ oder $K_2CO_3$) oder einem Erdalkalicarbonat (z.B. $MgCO_3$) oder einem Metallhydrid (z.B. NaH oder LiH) mit einer Verbindung der Formel HS-E, worin E z.B. Methyl, Ethyl, Isopropyl, Dodecyl oder Benzyl bedeuten kann, in einem inerten Lösungsmittel.

Die Herstellung der Verbindung der Formel VI kann auch in einem Eintopfverfahren über die Salze der Verbindung der Formel VIII erfolgen. Dabei wird die Verbindung IV in einem inerten, vorzugsweise dipolaren aprotischen Lösungsmittel, wie z.B. Dimethylsulfoxid, Dimethylformamid oder N-Methylpyrrolidon, in Gegenwart von zwei Aequivalenten einer Base, wie beispielsweise einem Carbonat (z.B. $Na_2CO_3$ oder $K_2CO_3$) oder einem Metallhydrid (z.B. NaH oder LiH) mit einer Verbindung der Formel HS-E, worin E ein unsubstituierter oder substituierter $C_1$-$C_{12}$-Alkylrest bedeutet (z.B. Methyl, Ethyl, Isopropyl, Dodecyl) oder Benzyl umsetzt und anschliessend mit einem Alkylierungsmittel R-L′, worin L′ eine Abgangsgruppe, wie z.B. Halogen, vorzugsweise Brom oder Iod, oder $-OSO_2R$ bedeutet, wobei R-L′ z.B. Dimethylsulfat sein kann, zur Verbindung der Formel VI verestert.

Der erhaltene 3-Mercapto-2-nitrobenzoesäureester der Form VI wird dann einer katalytischen oder metallischen Reduktion unterworfen. Bei der katalytischen Reduktion können beispielsweise Raney-Nickel sowie Palladium-, Platin- oder Rhodium-Katalysatoren verwendet werden; die Reduktion kann bei Normaldruck oder leicht erhöhtem Druck bei Temperaturen von 0° bis 150°C durchgeführt werden. Als Lösungsmittel kommen z.B. Tetrahydrofuran oder Dioxan in Frage.

Bei der metallischen Reduktion können z.B. Eisen/Salzsäure, Zinn/Salzsäure, Zink/Essigsäure als reduzierende Agentien verwendet werden; die Reduktion kann bei Temperaturen von 0° bis 120°C durchgeführt werden. Dabei können als Lösungsmittel Wasser, Alkohole, wie z.B. Methanol, Ethanol, n-Propanol oder iso-Propanol oder auch Tetrahydrofuran oder Dioxan eingesetzt werden. Auch Gemische der oben erwähnten Lösungsmittel kommen bei dieser Reaktion in Frage.

Die Verbindungen der Formel VII mit E = H können auch durch Hydrolyse der entsprechenden Amino-benzthiazole der Formel IX hergestellt werden

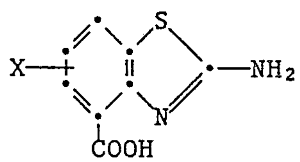

(IX)

die wiederum aus einer Aminobenzosäure der Formel X,

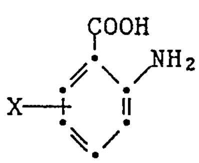

(X)

durch Umsetzung mit Thiocyanaten erhalten werden (siehe z.B. Organic Synthesis, Coll. Vol. 3, 76).

In den vorstehend beschriebenen Verfahren sind unter Basen, wenn nicht speziell angegeben, sowohl anorganische als auch organische Basen zu verstehen. Dazu zählen als anorganische Basen beispielsweise

Hydroxide, Oxide, Carbonate und Hydride des Lithiums, Natriums, Kaliums, Calciums und Bariums, sowie Alkaliamide, wie z.B. $NaNH_2$ oder Alkyllithiumverbindungen, wie z.B. n-Butyllithium. Als organische Basen sind beispielsweise zu nennen: Amine, insbesondere tertiäre Amine wie z.B. Trimethylamin, Triethylamin, Tripropylamin, Tributylamin, Tribenzylamin, Tricyclohexylamin, Triamylamin, Trihexylamin, N,N-Dimethylanilin, N,N-Dimethyltoluidin, N,N-Dimethyl-p-amino-pyridin, N-Methylpyrrolidin, N-Methyl-piperidin, N-Methyl-pyrrolidin, N-Methylimidazol, N-Methylpyrrol, N-Methylmorpholin, N-Methylhexamethylenimin, Pyridin, Chinolin, alpha-Picolin, β-Picolin, Isochinolin, Pyrimidin, Acridin, N,N,N′,N′-Tetra-methylethylendiamin, N,N,N′,N′-Tetraethylethylendiamin, Chinoxalin, N-Propyldiisopropylamin, N,N-Dimethylcyclohexylamin, 2,6-Lutidin, 2,4-Lutidin oder Triethylendiamin.

Als inerte Lösungsmittel sind in Anpassung an die jeweiligen Reaktionsbedingungen beispielsweise zu nennen:

Halogenkohlenwasserstoffe, insbesondere Chlorkohlenwasserstoffe, wie z.B. Tetrachorethylen, Tetrachlorethan, Dichlorpropan, Methylenchlorid, Dichlorbutan, Chloroform, Chlornaphthalin, Dichlornaphthalin, Tetrachlorkohlenstoff, Trichlorethan, Trichlorethylen, Pentachlorethan, Difluorbenzol, 1,2-Dichlorethan, 1,1-Dichlorethan, 1,2-cis-Dichlorethylen, Chlorbenzol, Fluorbenzol, Brombenzol, Jodbenzol, Dichlorbenzol, Dibrombenzol, Chlortoluol, Trichlorbenzol; Ether, wie z.B. Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, Dichlordiethylether, Methylcellosolve; Alkohole; wie z.B. Methanol, Ethanol, n-Propanol, iso-Propanol, n-Butanol; iso-Butanol; Nitrokohlenwasserstoffe wie z.B. Nitromethan, Nitroethan, Nitrobenzol, Chlornitrobenzol, o-Nitrotoluol; Nitrile wie z.B. Acetonitril, Butyro nitril, Isobutyronitril, Benzonitril, m-Chlorbenzonitril; aliphatische oder cycloaliphatische Kohlenwasserstoffe, wie z.B. Heptan, Pinan, Nonan, Cymol, Benzinfraktionen innerhalb eines Siedepunktinvervalls von 70° bis 190° C, Cyclohexan, Methylcyclohexan, Dekalin, Petrolether, Hexan, Ligroin, Trimethylpentan, Trimethylpentan, 2,3,3-Trimethylpentan, Octan; Ester wie z.B. Ethylacetat, Acetessigester, Isobutylacetat; Amide, wie z.B. Formamid, Methylformamid, Dimethylformamid; Ketone, wie Aceton, Methylethylketon, gegebenenfalls auch Wasser. Auch Gemische der genannten Lösungs- und Verdünnungsmittel kommen in Betracht.

Die vorstehend beschriebenen Herstellungsverfahren basieren, soweit nicht neu, auf bekannten Synthesemethoden, die der folgenden Literatur entnommen werden können:

The Chemistry of Heterocyclic Compounds with Nitrogen and Sulfur or Nitrogen, Sulfur and Oxygen, Interscience Publ., New York 1952; P. Kirby et al. J. Chem. Soc. (C) 321 (1967) und 2250 (1970) und 3994 (1971); FR-PS 1 541 415; Deutsche Offenlegungsschriften 2 400 887 und 2 504 383; SU-PS 400 574 [Chem. Abstr. 80(9)47661 h].

Bei den Verbindungen der Formeln I, If, II und VII handelt es sich um neue Stoffe, während die Verbindungen der Formeln IV, V und VI teilweise neu sind. Die neuen Verbindungen stellen einen Teil der vorliegenden Erfindung dar.

Die Wirkstoffe der Formel I werden in der Regel mit Aufwandmengen von 0,05 bis 4 kg/ha insbesondere 0,1 bis 1 kg/ha erfolgreich eingesetzt.

Die Verbindung der Formel I sind Pflanzenwachstumsregulatoren. Pflanzenwachstumsregulatoren sind Substanzen, die in/an der Pflanze agronomisch erwünschte biochemische und/oder physiologische und/oder morphologische Veränderungen bewirken. Ein Wachstumsregulator kann nach der bisherigen Erfahrung mehrere verschiedene Wirkungen haben, je nach Applikationszeitpunkt, Dosierung, Applikationsart oder Umweltbedingungen. Pflanzenwuchsregulierende Substanzen können zum Beispiel das vegetative Wachstum von Pflanzen hemmen. Diese Art der Wirkung ist von Interesse auf Rasenflächen, im Zierpflanzenbau, in Obstplantagen, an Strassenböschungen, auf Sport- und Industrieanlagen, aber auch bei der gezielten Hemmung von Nebentrieben wie bei Tabak. Im Ackerbau für die Hemmung des vegetativen Wachstums bei Getreide über eine Halmverstärkung zu reduziertem Lager, ähnlich agronomische Wirkung erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, so dass die Konkurrenz gegenüber der Hauptkultur ausgeschaltet ist, die agronomisch positiven Effekte wie Erosionsverhinderung, Stickstoffbindung und Bodenlockerung aber erhalten bleiben.

Durch Pflanzenwachstumsregulatoren kann der Ertrag quantitativ (z.B. Latexfluss) oder qualitativ (z.B. Zuckergehalt) beeinflusst werden, die Apikaldominanz kann gebrochen und die Seitentriebbildung gefördert werden (z.B. Zierpflanzen), Blüten- und Fruchtfall können gefördert werden (z.B. Ausdünnen bei Obstbäumen zur Alternanzbrechung, Fruchtabszission bei Oliven zur mechanischen Ernte), die Fruchtreife kann mit Wachstumsregulatoren harmonisiert, beschleunigt oder verzögert werden (z.B. Kapselöffnen bei Baumwolle, Reifung von Tomaten oder Bananen).

Mit Wachstumsregulatoren können Pflanzen gegen Umweltstress wie Trockenheit, Kälte oder Bodensalzgehalt resistenter gemacht werden. Schliesslich kann mit Pflanzenwachstumsregulatoren die Entblätterung von Kulturpflanzen zeitlich gezielt induziert werden, so dass die mechanische Ernte in Kulturen wie Baumwolle, Kartoffeln oder Reben erleichtert bzw. ermöglicht wird.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet.

Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen porose Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethanol Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1979.
Dr. Helmut Stache "Tensid Taschenbuch"

Carl Hanser Verlag, München/Wien 1981.

Die pflanzen- und wuchsregulierenden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

| | |
|---|---|
| Wirkstoff der Formel I: | 1 bis 20 % bevorzugt 5 bis 10 % |
| oberflächenaktives Mittel: | 5 bis 30 %,vorzugsweise 10 bis 20 % |
| flüssiges Trägermittel: | 50 bis 94 %,vorzugsweise 70 bis 85 %. |

Stäube:

| | |
|---|---|
| Wirkstoff der Formel I: | 0,1 bis 10 %, vorzugsweise 0,1 bis 1 % |
| festes Trägermittel: | 99,9 bis 90 %, vorzugsweise 99,9 bis 99 %. |

Suspensions-Konzentrate:

| | |
|---|---|
| Wirkstoff der Formel I: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

Benetzbare Pulver:

| | |
|---|---|
| Wirkstoff der Formel I: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

Granulate:

| | |
|---|---|
| Wirkstoff der Formel I: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung von 5-Chlor-benzothiadiazol-4-carbonsäuremethylester

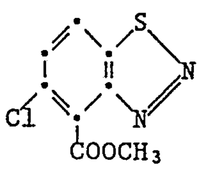

Eine Mischung von 5,70 g (0,0185 mol) 2-Amino-3-benzylthio-6-chlor-benzoesäuremethylester in 25 ml konzentrierter Salzsäure und 11 ml Wasser wird bei Raumtemperatur gut gerührt. Dann werden bei -10°C langsam 1,30 g Natriumnitrit in 5 ml Wasser zugetropft. Dabei findet eine Umfällung statt. Man lässt 16 Std. bei 5°C ausrühren, gibt dann etwas Hexan/Essigsäureethylester hinzu und filtriert den erhaltenen Niederschlag ab. Nach dem Waschen mit Wasser und anschliessendem Trocknen erhält man 3,6 g (85 %) Titelprodukt; Smp. 126-128°C.

Der als Ausgangsprodukt benötigte 2-Amino-3-benzylthiobenzoesäuremethyl ester wird wie folgt hergestellt:

a) Herstellung von 3-Benzylthio-6-chlor-2-nitro-benzoesäuremethylester

81,6 g (0,585 mol) Kaliumcarbonat werden zu 2,5 l 80 % Methanol gegeben und bei Raumtemperatur 64,0 ml (0,544 mol) Benzylmercaptan zugetropft. Darauf werden während 2 Stunden 160 g (0,544 mol) 3-Brom-6-chlor-2-nitro-benzoesäuremethylester zudosiert. Nach einiger Zeit bildet sich ein gelber Niederschlag. Das Reaktionsgut wird 16 Stunden bei Raumtemperatur gerührt und anschliessend filtriert. Der Niederschlag wird mit Wasser gewaschen, leicht getrocknet und in 80 % Methanol umkristallisiert. Ausbeute: 103,5g (56 %); Smp. 93°C.

b) Herstellung von 2-Amino-3-benzylthio-6-chlorbenzoesäuremethylester

16,9 g (0,05 mol) 3-Benzylthio-6-chlor-2-nitro-benzoesäuremethylester werden in 170 ml Tetrahydrofuran gelöst und in Gegenwart von 13 g Raney-Nickel bei Normaldruck und bei einer Temperatur von 20 bis 25°C hydriert. Anschliessend wird der Katalysator abfiltriert und das Filtrat eingeengt (Oel).

Beispiel 2: Herstellung von 1,2,3-Benzothiadiazol-4-carbonsäure-methylester

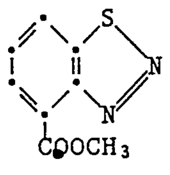

Zu 0,90 g (0,0033 mol) 2-Amino-3-benzylthiobenzoesäuremethylester werden 13,2 ml konzentrierter Salzsäure und 6 ml Wasser gegeben und gut gerührt. Dann wird bei -5 bis -10°C langsam eine Lösung von 0,25 g Natriumnitrit in 1 ml Wasser zudosiert. Anschliessend wird zwei Stunden bei -5°C und zwei Stunden bei Raumtemperatur gerührt. Darauf wird das Reaktionsgemisch auf Eis/Wasser gegeben und zweimal mit Essigsäureethylester extrahiert. Die organische Phase wird mit Wasser gewaschen, mit Magnesiumsulfat getrocknet, filtriert und eingedampft. Der hellgelbe Kristallbrei wird in Hexan verrieben, abfiltriert und die erhaltenen Kristalle getrocknet. Ausbeute: 0,50 g (78 %); Smp. 95-96°C.

Beispiel 3: Herstellung von 5-Chlor-1,2,3-benzothiadiazol-4-carbonsäurebenzylester

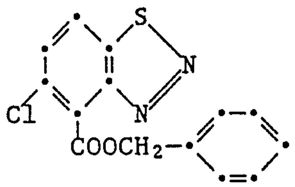

Eine Lösung von 1,30 g (0,012 mol) Benzylalkohol, 1,50 g (0,015 mol) Triethylamin und 50 ml Essigsäureethylester wird auf 15°C gekühlt, dann langsam mit 11 ml einer Lösung von 0,011 mol 5-Chlor-1,2,3-benzothiadiazol-4-carbonsäurechlorid in Essigsäureethylester versetzt und 5 Stun den bei Raumtemperatur gerührt. Das Reaktionsgemisch wird auf Eis/Wasser gegossen, die Phasen getrennt und die wässrige Phase noch zweimal mit Essigsäureethylester extrahiert. Die Extrakte werden mit Wasser gewaschen, mit Magnesiumsulfat getrocknet, filtriert und eingeengt. Nach der Reinigung durch eine kurze Kieselgelsäule (Lösungsmittel: Essigsäureethylester/Hexan) erhält man 2,9g (87 %) Produkt; Smp. 99-101°C.

Das als Ausgangsprodukt benötigte 5-Chlor-1,2,3-benzothiadiazol-4-carbonsäurechlorid wird wie folgt hergestellt:

a) Herstellung von 5-Chlor-1,2,3-benzothiadiazol-4-carbonsäure

19,5g (0,085 mol) 5-Chlor-1,2,3benzothiadiazol-4-carbonsäuremethylester werden in 165 ml Dioxan gelöst, mit 52 ml 2N Natronlauge versetzt und 4 Stunden bei 40°C gerührt. Nach dem Abkühlen werden 52 ml 2N Salzsäure zugegeben und das Gemisch mit Eis/Wasser versetzt. Der erhaltene Niederschlag wird filtriert, mit Wasser gewaschen und getrocknet.
Ausbeute: 18 g (98 %); Smp. 211-212°C.

b) Herstellung von 5-Chlor-1,2,3-benzothiadiazol-4-carbonsäure

12,0g (0,041 mol) 2-Amino-3-benzylmercapto-6-chlor-benzoesäure werden in einer Lösung von 55 ml konzentrierter Salzsäure und 25 ml Wasser suspendiert und während 30 Minuten bei 50°C gerührt. Nach dem Abkühlen auf -5°C wird eine Lösung von 2,9g (0,042 mol) Natriumnitrit in 10 ml Wasser langsam zugetropft. Dann wird das Reaktionsgemisch 3 Std. bei der gleichen Temperatur gerührt und danach lässt man die Temperatur während 17 Std. auf Raumtemperatur ansteigen. Der erhaltene Niederschlag wird filtriert, mit Wasser gewaschen, in Hexan/Ether verrührt, nochmals filtriert und getrocknet. Ausbeute: 6,2g (70 %).

c) Herstellung von 5-Chlor-1,2,3-benzothiadiazol-4-carbonsäurechlorid

10,0g (0,047 mol) 5-Chlor-1,2,3-benzothiadiazol-4-carbonsäure werden in 100 ml Toluol (über Molekularsieb getrocknet) suspendiert. Dann werden 25 ml Toluol abdestilliert und anschliessend während 1 Std. 7,4 ml (0,102 mol) Thionylchlorid bei 90°C zugetropft. Man lässt 6 Std. bei der gleichen Temperatur weiter rühren. Nach dem Abdestillieren des Lösungsmittels am Rotationsverdampfer wird das erhaltene Produkt direkt weiter verwendet.

Beispiel 4: 5-Methoxy-1,2,3-benzothiadiazol-4-carbonsäure-methylester

5,5g (0,024 mol) 5-Chlor-1,2,3-benzothiadiazol-4-carbonsäure-methylester, 50 ml Methanol und 8,1 ml Natriummethylatlösung (5,4M) werden 48 Std. am Rückfluss gehalten. Das Reaktionsgemisch wird auf Eis/Wasser gegossen, der erhaltene Niederschlag mit Wasser gewaschen, getrocknet und aus Essigester/Hexan umkristallisiert. Ausbeute: 3,5g (70 %); Smp. 93-94°C.

In analoger Weise zu diesen Beispielen werden die in den Tabellen 1-3 aufgeführten Verbindungen hergestellt.

Tabelle 1

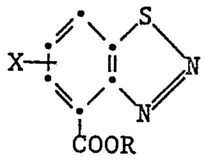

| Verb. Nr. | R | X | Physikal. Daten |
|---|---|---|---|
| 1.1 | H | H | |
| 1.2 | $CH_3$ | H | Smp. 95-96° |
| 1.3 | $C_2H_5$ | H | |
| 1.4 | n-$C_3H_7$ | H | |
| 1.5 | i-$C_3H_7$ | H | |
| 1.6 | n-$C_4H_9$ | H | |
| 1.7 | s-$C_4H_9$ | H | |
| 1.8 | t-$C_4H_9$ | H | |
| 1.9 | n-$C_5H_{11}$ | H | |
| 1.10 | n-$C_6H_{13}$ | H | |
| 1.11 | n-$C_8H_{17}$ | H | |
| 1.12 | 2-Bromethyl | H | |
| 1.13 | 2-Chlorethyl | H | |
| 1.14 | 2-Fluorethyl | H | |
| 1.15 | 2-Cyanoethyl | H | |
| 1.16 | 2-Methoxyethyl | H | |
| 1.17 | 2-n-Butoxyethyl | H | |
| 1.18 | 2-Allyloxyethyl | H | |
| 1.19 | 2,2,2-Trichlorethyl | H | |
| 1.20 | 3-Aethoxypropyl | H | |
| 1.21 | 3-Acetylpropyl | H | |
| 1.22 | 3-Chlorpropyl(n) | H | |
| 1.23 | 3-Brompropyl(n) | H | |
| 1.24 | 1-Chlorprop-2-yl | H | |
| 1.25 | 1-Bromprop-2-yl | H | |
| 1.26 | 2,3-Dibrompropyl(n) | H | |
| 1.27 | 2-Nitroethyl | H | |
| 1.28 | Cyclopropylmethyl | H | |

Tabelle 1 (Fortsetzung)

| Verb. Nr. | R | X | Physikal. Daten |
|---|---|---|---|
| 1.29 | 1-Cyclopropyl-eth-1-yl | H | |
| 1.30 | Cyclohexylmethyl | H | |
| 1.31 | Cyclooctylmethyl | H | |
| 1.32 | 3-Phenylpropyl | H | |
| 1.33 | 2-Phenylethyl | H | |
| 1.34 | Benzyl | H | |
| 1.35 | 2-Chlorbenzyl | H | |
| 1.36 | 3-Chlorbenzyl | H | |
| 1.37 | 4-Chlorbenzyl | H | |
| 1.38 | 4-Methylbenzyl | H | |
| 1.39 | 4-Methoxybenzyl | H | |
| 1.40 | 4-Nitrobenzyl | H | |
| 1.41 | 2-(4-Methoxyphenyl)ethyl | H | |
| 1.42 | 2-Phenoxyethyl | H | |
| 1.43 | 2-(4-Chlorphenoxy)ethyl | H | |
| 1.44 | Allyl | H | |
| 1.45 | 4-Pentenyl | H | |
| 1.46 | 2-Propinyl | H | |
| 1.47 | 3-Hexinyl | H | |
| 1.48 | 3-Chlor-but-2-enyl | H | |
| 1.49 | Cyclopropyl | H | |
| 1.50 | Cyclopentyl | H | |
| 1.51 | Cyclooctyl | H | |
| 1.52 | Phenyl | H | |
| 1.53 | 2-Chlorphenyl | H | |
| 1.54 | 3-Bromphenyl | H | |
| 1.55 | 3,4-Dichlorphenyl | H | |
| 1.56 | 4-Chlor-2-methyl-phenyl | H | |
| 1.57 | 4-t-Butylphenyl | H | |
| 1.58 | 3-Nitrophenyl | H | |
| 1.59 | 4-Nitrophenyl | H | |
| 1.60 | 3-Cyanophenyl | H | |

Tabelle 1 (Fortsetzung)

| Verb. Nr. | R | X | Physikal. Daten |
|---|---|---|---|
| 1.61 | 3-Trifluormethylphenyl | H | |
| 1.62 | 3-N,N-Dimethylaminophenyl | H | |
| 1.63 | 2-Methoxycarbonyl-phenyl | H | |
| 1.64 | 3-Jod-prop-2-in-yl | H | |
| 1.65 | $-CH_2-COOCH_3$ | H | |
| 1.66 | $-CH_2-COOC_2H_5$ | H | |
| 1.67 | $-CH(CH_3)-COOCH_3$ | H | |
| 1.68 | $-CH(CH_3)-COOC_2H_5$ | H | |
| 1.69 | $-CH_2CH_2N(CH_3)_2$ | H | |
| 1.70 | 3-N,N-Dimethylaminopropyl | H | |
| 1.71 | $-N=C(CH_3)_2$ | H | |
| 1.72 | -N=(cyclohexyl) | H | |
| 1.73 | -N=(p-tert.butyl-cyclohexyl) | H | |
| 1.74 | $-N=C(CH_3)CH_2OCH_3$ | H | |
| 1.75 | $-N=C(CH_3)C_2H_5$ | H | |
| 1.76 | $-N=C(CN)-C_6H_5$ | H | |
| 1.77 | $-N=C(C_2H_5)C_2H_5$ | H | |
| 1.78 | $-N=C(CH_3)C_6H_5$ | H | |
| 1.79 | $-CH_2CH_2CH_2OH$ | H | |
| 1.80 | $-CH_2CH_2OH$ | H | |
| 1.81 | 3-Fluorbenzyl | H | |
| 1.82 | 4-Trifluorbenzyl | H | |
| 1.83 | Furan-2-ylethyl | H | |
| 1.84 | Pyridin-2-ylethyl | H | |
| 1.85 | Pyrrolidinoethyl | H | |
| 1.86 | Diaceton-D-glucos-3-yl | H | |
| 1.87 | 2-Fluorbenzyl | H | |
| 1.88 | 4-Fluorbenzyl | H | |
| 1.89 | 4-Methylphenyl | H | |
| 1.90 | 2-Methoxycarbonylphenyl | H | |

Tabelle 1 (Fortsetzung)

| Verb. Nr. | R | X | Physikal. Daten |
|---|---|---|---|
| 1.91 | 2-Carboxyphenyl | H | |
| 1.92 | Tetrahydrofuran-2-ylmethyl | H | |
| 1.93 | Tetrahydropyran-2-ylmethyl | H | |
| 1.94 | Tetrahydrofuran-3-ylmethyl | H | |
| 1.95 | 4-Methylpiperidinomethyl | H | |
| 1.96 | Furan-2-ylmethyl | H | |
| 1.97 | Thiophen-2-ylmethyl | H | |
| 1.98 | 1-Methylpyrrol-2-ylmethyl | H | |
| 1.99 | Imidazol-2-ylmethyl | H | |
| 1.100 | Imidazol-4-ylmethyl | H | |
| 1.101 | Pyridin-2-ylmethyl | H | |
| 1.102 | Pyridin-3-ylmethyl | H | |
| 1.103 | $CH_2Si(CH_3)_3$ | H | |
| 1.104 | $CH_2CH_2Si(CH_3)_3$ | H | |
| 1.105 | $CH_2P(O)(CH_3)OC_2H_5$ | H | |
| 1.106 | $CH_2P(O)(OCH_3)_2$ | H | |
| 1.107 | $CH_2CH_2P(O)(OC_2H_5)_2$ | H | |
| 1.108 | $CH_2CH_2P(O)(OCH_3)_2$ | H | |
| 1.109 | $CH_2P(O)(CH_3)OCH_3$ | H | |
| 1.110 | $CH(CH_3)P(O)(OCH_3)_2$ | H | |
| 1.111 | $Si(CH_3)_2C(CH_3)_2CH(CH_3)_2$ | H | |
| 1.112 | Na | H | |
| 1.113 | K | H | |
| 1.114 | $(HN(C_2H_5)_3)^{\oplus}$ | H | |
| 1.115 | $(H_2N(CH_2CH_2OH)_2)^{\oplus}$ | H | |
| 1.116 | $CH_2$-Napth-1'-yl | H | |
| 1.117 | $CH_2$-Naphth-2'-yl | H | |
| 1.118 | $CH_2CH_2$-Naphth-1'-yl | H | |
| 1.119 | 1-Phenethyl | H | |
| 1.120 | 2-Phenyl-prop-2-yl | H | |
| 1.121 | $CH_2CH_2CN$ | H | |
| 1.122 | 2,3-Dihydroxyprop-1'-yl | H | |
| 1.123 | $CH_2CH_2SCH_3$ | H | |

Tabelle 1 (Fortsetzung)

| Verb. Nr. | R | X | Physikal. Daten |
|---|---|---|---|
| 1.124 | 1,2,3,4-Di-O-isopropyliden-D-galactopyranos-6-yl | H | |
| 1.125 | 1,2,5,6-Di-O-isopropyliden-D-mannit-3-yl | H | |
| 1.126 | 1,2,5,6-Di-O-isopropyliden-α-D-allofuranos-3-yl | H | |
| 1.127 | D-Glucofuranos-3-yl | H | |
| 1.128 | D-Galactopyranos-6-yl | H | |
| 1.129 | D-Mannit-3-yl | H | |
| 1.130 | D-Allofuranos-4-yl | H | |
| 1.131 | Mannopyranos-1-yl | H | |
| 1.132 | 2-Methyl-D-glucosid-6-yl | H | |
| 1.133 | 1,2,5,6-Tetraacetyl-D-galactopyranos-3-yl | H | |
| 1.134 | 2,3,5-Tribenzylribofuranos-1-yl | H | |
| 1.135 | H | 5-Cl | Smp. 211-212° |
| 1.136 | $CH_3$ | 5-Cl | Smp. 126-128° |
| 1.137 | $C_2H_5$ | 5-Cl | Smp. 107-108° |
| 1.138 | $n-C_3H_7$ | 5-Cl | |
| 1.139 | $i-C_3H_7$ | 5-Cl | |
| 1.140 | $n-C_4H_9$ | 5-Cl | Smp. 53-54° |
| 1.141 | $s-C_4H_9$ | 5-Cl | |
| 1.142 | $t-C_4H_9$ | 5-Cl | |
| 1.143 | $n-C_5H_{11}$ | 5-Cl | |
| 1.144 | $n-C_6H_{13}$ | 5-Cl | |
| 1.145 | $n-C_8H_{17}$ | 5-Cl | |
| 1.146 | 2-Bromethyl | 5-Cl | |
| 1.147 | 2-Chlorethyl | 5-Cl | |
| 1.148 | 2-Fluorethyl | 5-Cl | |
| 1.149 | 2-Cyanoethyl | 5-Cl | |
| 1.150 | 2-Methoxyethyl | 5-Cl | |
| 1.151 | 2-n-Butoxyethyl | 5-Cl | |
| 1.152 | 2-Allyloxyethyl | 5-Cl | |
| 1.153 | 2,2,2-Trichlorethyl | 5-Cl | |
| 1.154 | 3-Aethoxypropyl | 5-Cl | |

Tabelle 1 (Fortsetzung)

| Verb. Nr. | R | X | Physikal. Daten |
|---|---|---|---|
| 1.155 | 3-Acetylpropyl | 5-Cl | |
| 1.156 | 3-Chlorpropyl(n) | 5-Cl | |
| 1.157 | 3-Brompropyl(n) | 5-Cl | |
| 1.158 | 1-Chlorprop-2-yl | 5-Cl | |
| 1.159 | 1-Bromprop-2-yl | 5-Cl | |
| 1.160 | 2,3-Dibrompropyl(n) | 5-Cl | |
| 1.161 | 2-Nitroethyl | 5-Cl | |
| 1.162 | Cyclopropylmethyl | 5-Cl | |
| 1.163 | 1-Cyclopropyl-eth-1-yl | 5-Cl | |
| 1.164 | Cyclohexylmethyl | 5-Cl | |
| 1.165 | Cyclooctylmethyl | 5-Cl | |
| 1.166 | 3-Phenylpropyl | 5-Cl | |
| 1.167 | 2-Phenylethyl | 5-Cl | |
| 1.168 | Benzyl | 5-Cl | Smp. 99-101° |
| 1.169 | 2-Chlorbenzyl | 5-Cl | Smp. 107° |
| 1.170 | 3-Chlorbenzyl | 5-Cl | |
| 1.171 | 4-Chlorbenzyl | 5-Cl | |
| 1.172 | 4-Methylbenzyl | 5-Cl | |
| 1.173 | 4-Methoxybenzyl | 5-Cl | |
| 1.174 | 4-Nitrobenzyl | 5-Cl | |
| 1.175 | 2-(4-Methoxyphenyl)ethyl | 5-Cl | |
| 1.176 | 2-Phenoxyethyl | 5-Cl | |
| 1.177 | 2-(4-Chlorphenoxy)ethyl | 5-Cl | |
| 1.178 | Allyl | 5-Cl | |
| 1.179 | 4-Pentenyl | 5-Cl | |
| 1.180 | 2-Propinyl | 5-Cl | Smp. 137-138° |
| 1.181 | 3-Hexinyl | 5-Cl | |
| 1.182 | 3-Chlor-but-2-enyl | 5-Cl | |
| 1.183 | Cyclopropyl | 5-Cl | |
| 1.184 | Cyclopentyl | 5-Cl | |
| 1.185 | Cyclooctyl | 5-Cl | |
| 1.186 | Phenyl | 5-Cl | |
| 1.187 | 2-Chlorphenyl | 5-Cl | |

Tabelle 1 (Fortsetzung)

| Verb. Nr. | R | X | Physikal. Daten |
|---|---|---|---|
| 1.188 | 3-Bromphenyl | 5-Cl | |
| 1.189 | 3,4-Dichlorphenyl | 5-Cl | |
| 1.190 | 4-Chlor-2-methyl-phenyl | 5-Cl | |
| 1.191 | 4-t-Butylphenyl | 5-Cl | |
| 1.192 | 3-Nitrophenyl | 5-Cl | |
| 1.193 | 4-Nitrophenyl | 5-Cl | |
| 1.194 | 3-Cyanophenyl | 5-Cl | |
| 1.195 | 3-Trifluormethylphenyl | 5-Cl | |
| 1.196 | 3-N,N-Dimethylaminophenyl | 5-Cl | |
| 1.197 | 2-Methoxycarbonyl-phenyl | 5-Cl | |
| 1.198 | 3-Jod-prop-2-in-yl | 5-Cl | |
| 1.199 | $-CH_2-COOCH_3$ | 5-Cl | |
| 1.200 | $-CH_2-COOC_2H_5$ | 5-Cl | |
| 1.201 | $-CH(CH_3)-COOCH_3$ | 5-Cl | |
| 1.202 | $-CH(CH_3)-COOC_2H_5$ | 5-Cl | |
| 1.203 | $-CH_2CH_2N(CH_3)_2$ | 5-Cl | |
| 1.204 | 3-N,N-Dimethylaminopropyl | 5-Cl | |
| 1.205 | $-N=C(CH_3)_2$ | 5-Cl | Smp. 78-79° |
| 1.206 | -N=(Cyclohexyl) | 5-Cl | |
| 1.207 | -N=(p-tBu-cyclohexyl) | 5-Cl | |
| 1.208 | $-N=C(CH_3)CH_2OCH_3$ | 5-Cl | |
| 1.209 | $-N=C(CH_3)C_2H_5$ | 5-Cl | |
| 1.210 | $-N=C(CN)-C_6H_5$ | 5-Cl | |
| 1.211 | $-N=C(CH_3)-C_6H_5$ | 5-Cl | |
| 1.212 | $-N=C(C_2H_5)_2$ | 5-Cl | |
| 1.213 | $-CH_2CH_2CH_2OH$ | 5-Cl | |
| 1.214 | $-CH_2CH_2OH$ | 5-Cl | |
| 1.215 | 3-Fluorbenzyl | 5-Cl | |
| 1.216 | 4-Trifluorbenzyl | 5-Cl | |
| 1.217 | Furan-2-yl-ethyl | 5-Cl | |
| 1.218 | Pyridin-2-yl-ethyl | 5-Cl | |

Tabelle 1 (Fortsetzung)

| Verb. Nr. | R | X | Physikal. Daten |
|---|---|---|---|
| 1.219 | Pyrrolidinoethyl | 5-Cl | |
| 1.220 | Diaceton-D-glucos-3-yl | 5-Cl | |
| 1.221 | 2-Fluorbenzyl | 5-Cl | |
| 1.222 | 4-Fluorbenzyl | 5-Cl | |
| 1.223 | 4-Methylphenyl | 5-Cl | |
| 1.224 | 2-Methoxycarbonylphenyl | 5-Cl | |
| 1.225 | 2-Carboxyphenyl | 5-Cl | |
| 1.226 | Tetrahydrofuran-2-yl-methyl | 5-Cl | |
| 1.227 | Tetrahydrofuran-3-yl-methyl | 5-Cl | |
| 1.228 | Furan-3-yl-methyl | 5-Cl | |
| 1.229 | 4-Methyl-piperidino-methyl | 5-Cl | |
| 1.230 | Furan-2-yl-methyl | 5-Cl | |
| 1.231 | Thiophen-2-yl-methyl | 5-Cl | |
| 1.232 | 1-Methyl-pyrrol-2-yl-methyl | 5-Cl | |
| 1.233 | Pyrimidin-2-yl-methyl | 5-Cl | |
| 1.234 | Pyrimidin-4-yl-methyl | 5-Cl | |
| 1.235 | Pyridin-2-yl-methyl | 5-Cl | |
| 1.236 | Pyrimidin-3-yl-methyl | 5-Cl | |
| 1.237 | $CH_2Si(CH_3)_3$ | 5-Cl | |
| 1.238 | $CH_2CH_2Si(CH_3)_3$ | 5-Cl | Smp. 102° |
| 1.239 | $CH_2P(O)(CH_3)OC_2H_5$ | 5-Cl | |
| 1.240 | $CH_2P(O)(OCH_3)_2$ | 5-Cl | |
| 1.241 | $CH_2CH_2P(O)(OC_2H_5)_2$ | 5-Cl | |
| 1.242 | $CH_2CH_2P(O)(OCH_3)_2$ | 5-Cl | Smp. 75° |
| 1.243 | $CH_2P(O)(CH_3)OCH_3$ | 5-Cl | |
| 1.244 | $CH(CH_3)P(O)(OCH_3)_2$ | 5-Cl | |
| 1.245 | $Si(CH_3)_2C(CH_3)_2CH(CH_3)_2$ | 5-Cl | |
| 1.246 | Na | 5-Cl | |
| 1.247 | K | 5-Cl | |
| 1.248 | $(HN(C_2H_5)_3)^{\oplus}$ | 5-Cl | |
| 1.249 | $(H_2N(CH_2CH_2OH)_2)^{\oplus}$ | 5-Cl | |
| 1.250 | $CH_2$-Napth-1'-yl | 5-Cl | |
| 1.251 | $CH_2$-Naphth-2'-yl | 5-Cl | |

Tabelle 1 (Fortsetzung)

| Verb. Nr. | R | X | Physikal. Daten |
|---|---|---|---|
| 1.252 | $CH_2CH_2$-Naphth-1'-yl | 5-Cl | |
| 1.253 | 1-Phenethyl | 5-Cl | |
| 1.254 | 2-Phenyl-prop-2-yl | 5-Cl | |
| 1.255 | $CH_2CH_2CN$ | 5-Cl | |
| 1.256 | 2,3-Dihydroxyprop-1'-yl | 5-Cl | |
| 1.257 | $CH_2CH_2SCH_3$ | 5-Cl | |
| 1.258 | 1,2,3,4-Di-O-isopropyliden-D-galactopyranos-6-yl | 5-Cl | |
| 1.259 | 1,2,5,6-Di-O-isopropyliden-D-mannit-3-yl | 5-Cl | |
| 1.260 | 1,2,5,6-Di-O-isopropyliden-α-D-allofuranos-3-yl | 5-Cl | |
| 1.261 | D-Glucofuranos-3-yl | 5-Cl | |
| 1.262 | D-Galactopyranos-6-yl | 5-Cl | |
| 1.263 | D-Mannit-3-yl | 5-Cl | |
| 1.264 | D-Allofuranos-4-yl | 5-Cl | |
| 1.265 | Mannopyranos-1-yl | 5-Cl | |
| 1.266 | 2-Methyl-D-glucosid-6-yl | 5-Cl | |
| 1.267 | 1,2,5,6-Tetraacetyl-D-galactopyranos-3-yl | 5-Cl | |
| 1.268 | 2,3,5-Tribenzylribofuranos-1-yl | 5-Cl | |
| 1.269 | Cyclohexyl | 5-Cl | Smp. 93-94° |
| 1.270 | H | 5-F | |
| 1.271 | $CH_3$ | 5-F | |
| 1.272 | $C_2H_5$ | 5-F | |
| 1.273 | $CH_2C_6H_5$ | 5-F | |
| 1.274 | $CH_3$ | 6-F | |
| 1.275 | $CH(CH_3)_2$ | 7-F | |
| 1.276 | $CH_3$ | 5-$CH_3$ | |
| 1.277 | $CH_3$ | 5-Br | |
| 1.278 | $CH_3$ | 5-$OCH_3$ | Smp. 93-94° |
| 1.279 | 2,6-Dimethyl-morpholino-ethyl | 5-Cl | |
| 1.280 | Piperidinoethyl | 5-Cl | |

Tabelle 1 (Fortsetzung)

| Verb. Nr. | R | X | Physikal. Daten |
|---|---|---|---|
| 1.281 | Pyrrolidino-ethyl | 5-F | |
| 1.282 | Pyridin-3-yl-methyl | 5-F | |
| 1.283 | Pyridin-4-yl-methyl | 5-Cl | |
| 1.284 | $CH_3$ | 6-Cl | |
| 1.285 | H | 6-Cl | |
| 1.286 | $CH_3$ | 6-Cl | |

Tabelle 2

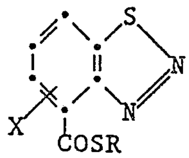

| Verb. Nr. | R | X | Physikal. Daten |
|---|---|---|---|
| 2.1 | $CH_3$ | H | |
| 2.2 | $C_2H_5$ | H | |
| 2.3 | $C_3H_7(n)$ | H | |
| 2.4 | $C_4H_9(n)$ | H | |
| 2.5 | Benzyl | H | |
| 2.6 | Phenyl | H | |
| 2.7 | 4-Chlorphenyl | H | |
| 2.8 | $CH_2COOCH_3$ | H | |
| 2.9 | $CH_2COOC_2H_5$ | H | |
| 2.10 | 4-Methylphenyl | H | |
| 2.11 | $CH_3$ | 5-Cl | Smp. 144-146° |
| 2.12 | $C_2H_5$ | 5-Cl | |
| 2.13 | $C_3H_7(n)$ | 5-Cl | |
| 2.14 | $C_4H_9(n)$ | 5-Cl | |
| 2.15 | Benzyl | 5-Cl | |
| 2.16 | Phenyl | 5-Cl | |
| 2.17 | 4-Chlorphenyl | 5-Cl | |
| 2.18 | $CH_2COOCH_3$ | 5-Cl | Smp. 100° |
| 2.19 | $CH_2COOC_2H_5$ | 5-Cl | |
| 2.20 | 4-Methylphenyl | 5-Cl | |
| 2.21 | $CH_3$ | 5-F | |
| 2.22 | $C_2H_5$ | 5-F | |
| 2.23 | $C_3H_7(n)$ | 5-F | |
| 2.24 | $C_4H_9(n)$ | 5-F | |
| 2.25 | Benzyl | 5-F | |
| 2.26 | Phenyl | 5-F | |
| 2.27 | 4-Chlorphenyl | 5-F | |
| 2.28 | $CH_2COOCH_3$ | 5-F | |
| 2.29 | $CH_2COOC_2H_5$ | 5-F | |
| 2.30 | 4-Methylphenyl | 5-F | |
| 2.31 | $CH_3$ | 6-F | |

Tabelle 2 (Fortsetzung)

| Verb. Nr. | R | X | Physikal. Daten |
|---|---|---|---|
| 2.32 | $C_2H_5$ | 5-$CH_3$ | |
| 2.33 | $C_3H_7$(n) | 5-Br | |
| 2.34 | $C_4H_9$(n) | 5-$OCH_3$ | |
| 2.35 | Benzyl | 7-F | |
| 2.36 | $CH_3$ | 5-$SCH_3$ | Smp. 70° |

Tabelle 3 (Zwischenprodukte)

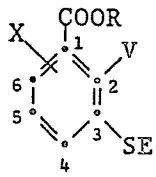

| Verb. Nr. | X | V | R | E | Physikal. Daten |
|---|---|---|---|---|---|
| 3.1 | H | $NO_2$ | H | $CH_3$ | |
| 3.2 | H | $NO_2$ | $CH_3$ | $CH(CH_3)_2$ | |
| 3.3 | H | $NO_2$ | $C_2H_5$ | $C_2H_5$ | |
| 3.4 | H | $NO_2$ | $CH_3$ | $CH_2C_6H_5$ | |
| 3.5 | H | $NO_2$ | $CH_3$ | $(CH_2)_{11}CH_3$ | |
| 3.6 | H | $NO_2$ | $CH_3$ | H | |
| 3.7 | H | $NO_2$ | H | $CH_2C_6H_5$ | |
| 3.8 | H | $NH_2$ | H | $CH_3$ | |
| 3.9 | H | $NH_2$ | $CH_3$ | $CH(CH_3)_2$ | |
| 3.10 | H | $NH_2$ | $C_2H_5$ | $C_2H_5$ | |
| 3.11 | H | $NH_2$ | $CH_3$ | $CH_2C_6H_5$ | Oel |
| 3.12 | H | $NH_2$ | $CH_3$ | $(CH_2)_{11}CH_3$ | |
| 3.13 | H | $NH_2$ | $CH_3$ | H | |
| 3.14 | H | $NH_2$ | H | $CH_2C_6H_5$ | |
| 3.15 | 6-Cl | $NO_2$ | H | $CH_3$ | |
| 3.16 | 6-Cl | $NO_2$ | $CH_3$ | $CH(CH_3)_2$ | |
| 3.17 | 6-Cl | $NO_2$ | $C_2H_5$ | $C_2H_5$ | |
| 3.18 | 6-Cl | $NO_2$ | $CH_3$ | $CH_2C_6H_5$ | Smp. 93° |
| 3.19 | 6-Cl | $NO_2$ | $CH_3$ | $(CH_2)_{11}CH_3$ | Smp. 58-59° |
| 3.20 | 6-Cl | $NO_2$ | $CH_3$ | H | |
| 3.21 | 6-Cl | $NO_2$ | H | $CH_2C_6H_5$ | |
| 3.22 | 6-Cl | $NH_2$ | H | $CH_3$ | |
| 3.23 | 6-Cl | $NH_2$ | $CH_3$ | $CH(CH_3)_2$ | |
| 3.24 | 6-Cl | $NH_2$ | $C_2H_5$ | $C_2H_5$ | |
| 3.25 | 6-Cl | $NH_2$ | $CH_3$ | $CH_2C_6H_5$ | Oel |
| 3.26 | 6-Cl | $NH_2$ | $CH_3$ | $(CH_2)_{11}CH_3$ | |
| 3.27 | 6-Cl | $NH_2$ | $CH_3$ | H | |
| 3.28 | 6-Cl | $NH_2$ | H | $CH_2C_6H_5$ | |
| 3.29 | 6-Cl | $NH_2$ | $CH_2C_6H_5$ | $CH_2C_6H_5$ | |
| 3.30 | 6-F | $NO_2$ | H | $CH_2C_6H_5$ | |

Tabelle 3 (Fortsetzung)

| Verb. Nr. | X | V | R | E | Physikal. Daten |
|---|---|---|---|---|---|
| 3.31 | 6-F | $NO_2$ | $CH_3$ | $CH_2C_6H_5$ | |
| 3.32 | 6-F | $NO_2$ | $C_2H_5$ | $C_2H_5$ | |
| 3.33 | 6-F | $NO_2$ | $CH_3$ | $(CH_2)_{11}CH_3$ | |
| 3.34 | 6-F | $NO_2$ | H | $(CH_2)_{11}CH_3$ | |
| 3.35 | 6-F | $NH_2$ | H | $CH_2C_6H_5$ | |
| 3.36 | 6-F | $NH_2$ | $CH_3$ | $CH_2C_6H_5$ | |
| 3.37 | 6-F | $NH_2$ | $C_2H_5$ | $C_2H_5$ | |
| 3.38 | 6-F | $NH_2$ | $CH_3$ | $(CH_2)_{11}CH_3$ | |
| 3.39 | 6-F | $NH_2$ | H | $(CH_2)_{11}CH_3$ | |
| 3.40 | 6-Br | $NO_2$ | $CH_3$ | $(CH_2)_{11}CH_3$ | |
| 3.41 | 6-Br | $NH_2$ | $CH_3$ | $(CH_2)_{11}CH_3$ | |
| 3.42 | H | $NO_2$ | H | $(CH_2)_{11}CH_3$ | Smp. 123° |
| 3.43 | H | $NH_2$ | H | $(CH_2)_{11}CH_3$ | |

Formulierungsbeispiele:

Beispiel 5: Formulierungsbeispiele für Wirkstoffe der Formel I

(% = Gewichtsprozent)

a) Spritzpulver

| | a) | b) |
|---|---|---|
| Wirkstoff gemäss Tabellen 1 und 2 | 10 % | 1 % |
| Octylphenolpolyethylenglykolether (4-5 Mol EO) | 3 % | 3 % |
| Ca-Dodecylbenzolsulfonat | 3 % | 3 % |
| Ricinusölpolyglykolether (36 Mol EO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff gemäss Tabellen 1 und 2 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff gemäss Tabellen 1 und 2 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff gemäss Tabellen 1 und 2 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff gemäss Tabellen 1 und 2 | 40 % | 5 % |
| Ethylenglykol | 10 % | 10 % |
| Nonylphenolpolyethylenglykolether (15 Mol EO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 6: Wuchshemmung bei tropischen Bodenbedecker-Leguminosen (cover crops).

Die Versuchspflanzen (Psophocarpus palestris und Centrosema pubescens) werden in 4 cm - Torftöpfen mit Landerde (45%), Torf (45 %) und Zonolit (10 %) durch Stecklinge vermehrt. Die Anzucht erfolgt im Gewächshaus bei einer Tagestemperatur von 27 °C und einer Nachttemperatur von 23°C. Die Beleuchtungsdauer ist mindestens 14 Stunden/Tag bei einer Intensität von mindestens 7000 Lux.

Ca. 50 Tage nach Ansatz der Stecklinge erfolgt das Vertopfen in 13 cm-Töpfe, 4-5 Pflanzen/Topf. Nach weiteren 60 Tagen werden die Pflanzen auf ca. 15 cm Höhe zurückgeschnitten und mit einer wässrigen Spritzbrühe besprüht. Die Spritzbrühe wird aus einem 25 % formulierten Spritzpulver durch Verdünnen mit Wasser hergestellt; die Aufwandmengen betragen 0,3-3 kg/ha Wirkstoff bei 200 l/ha Wasser. 4 Wochen nach der Applikation wird der Neuzuwachs im Gewicht ermittelt und in % zum Durchschnitt der unbehandelten Kontrollen dargestellt. Die geprüften Verbindungen bewirken eine Reduzierung des Neuzuwachses von 10-60 %.

Beispiel 7: Wuchsregulierung bei Sojabohnen

Die Sojabohnen der Sorte Williams werden in 11 cm Tontöpfen mit Landerde (45 %) Torf (45 %) und Zonolit (10 %) angesät und in der Klimakammer bei einer Tagestemperatur von 24° C und einer Nachttemperatur von 19° C angezogen. Die Beleuchtungsdauer ist 16 Stunden pro Tag bei einer Intensität von ca. 350 Mikro-Einstein.

Ca. 24 Tage der Saat erfolgt das Umtopfen im 18 cm-Töpfe, 2 Pflanzen/Topf. Nach weiteren 12 Tagen und im Stadium 6-5 trifol. Blätter findet die Applikation mit Konzentrationsreihen von 10 bis 2000 g Wirkstoff/ha statt. Der Wirkstoff ist 25 %ig formuliert und wird in wässriger Spritzbrühe mit einer Wasseraufwandmenge von 200 l/ha gespritzt.

Ca. 4 Wochen nach der Applikation findet die Auswertung statt. Hierbei wird die Höhe des Neuzuwachses gemessen und in % zum Durchschnitt der unbehandelten Kontrollen dargestellt. Die geprüften Verbindungen bewirken eine Reduzierung des Neuzuwachses um 10 bis 80 %.

Beispiel 8: Wuchshemmung bei Getreide

Sommergeste der Sorte "Iban" wird in 15 cm-Kunststofftöpfen mit steriler Landerde angesät und in der Klimakammer bei einer Tagestemperatur von 10-15° C und einer Nachttemperatur von 5-10° C angezogen. Die Beleuchtungsdauer ist 13,5 Stunden pro Tag bei einer Intensität von ca. 25000 Lux.

Ca. 34 Tage der Saat und dem Ausdünnen auf 4 Pflanzen/Topf erfolgt die Applikation mit 0,3 bis 3 kg Wirkstoff/ha. Der Wirkstoff ist 25 %ig formuliert und wird in wässriger Spritzbrühe gesprüht. Die Wasseraufwandmenge ist 500 l/ha. Nach der Applikation werden die Pflanzen im Gewächshaus bei einer Tagestemperatur von mindestens 10° C aufgestellt. Die Beleuchtungsdauer ist mindestens 13,5 Stunden/Tag.

28 Tage nach der Behandlung findet die Auswertung statt. Hierbei wird die Höhe des Neuzuwachses in % zum Durchschnitt der unbehandelten Kontrollen dargestellt. Die geprüften Verbindungen bewirken eine Reduzierung des Neuzuwachses um 10 bis 80 %.

**Patentansprüche**

1. 1,2,3-Benzthiazol-4-carbonsäure-Derivate der Formel I

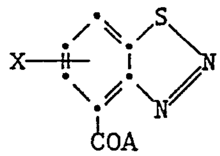

(I)

in welcher

X Wasserstoff, Halogen, $C_1$-$C_3$-Alkyl, $C_1$-$C_3$-Alkoxy oder $C_1$-$C_3$-Alkylthio,

A einen Rest -OR, -SR oder -ON = $C(R_1)R_2$,

R Wasserstoff, ein Metall- oder Ammoniumkation, einen unsubstituierten oder substituierten Alkyl-, Alkenyl-, Alkinyl-, Cycloalkyl- oder Arylrest, einen heterocyclischen Rest oder einen Monosaccharid-Rest,

$R_1$ und $R_2$ unabhängig voneinander je Wasserstoff, $C_1$-$C_6$-Alkyl, $C_2$-$C_8$-Alkoxyalkyl, CN, unsubstituiertes oder substituiertes Phenyl oder einen substituierten oder unsubstituierten Alkanoyl- oder Alkenoyl-Rest bedeuten und

$R_1$ und $R_2$ zusammen mit dem Kohlenstoffatom, an das sie gebunden sind, auch einen heterocyclischen oder einen carbocyclischen Rest mit 3-7 Ringatomen bilden können.

2. 1,2,3-Benzthiadiazol-4-carbonsäure-Derivate gemäss Anspruch 1 der Formel I

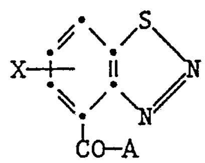

(I)

worin X Wasserstoff, Halogen, $C_1$-$C_3$-Alkyl, $C_1$-$C_3$-Alkoxy oder $C_1$-$C_3$-Alkylthio

A einen Rest -OR, -SR oder -ON = $C(R_1)R_2$ oder

n Null oder 1,

R Wasserstoff, ein Alkalimetall-, Erdalkalimetall, Aluminium-, Kupfer-, Nickel-, Mangan-, Zink-, Eisen- oder Silberkation, den Rest einer Base oder ein Ammoniumkation der Formel

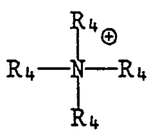

,

wobei die $R_4$ unabhängig voneinander durch Wasserstoff, $C_1$-$C_6$-Alkyl, $C_1$-$C_6$-Hydroxyalkyl, $C_2$-$C_6$-Alkoxyalkyl, $C_1$-$C_6$-Halogenalkyl, $C_3$-$C_6$-Alkenyl, Benzyl, Pyridinyl vertreten sein können, zwei $R_4$ können zusammen auch eine $C_3$-$C_7$-Alkylenkette bilden, die durch Sauerstoff unterbrochen sein kann,

R ist ferner $C_1$-$C_8$-Alkyl, durch Halogen, Cyano, Nitro, Hydroxy oder U-$C_1$-$C_3$-Alkyl substituiertes $C_1$-$C_8$-Alkyl, (T)-CO-$C_1$-$C_4$-Alkyl, (T)-COOH oder (T)-COO$C_1$-$C_4$-Alkyl, $C_2$-$C_6$-Alkenyl, durch Halogen substituiertes $C_3$-$C_6$-Alkenyl, $C_3$-$C_6$-Alkinyl, durch Halogen substituiertes $C_3$-$C_6$-Alkinyl, $(T)_n$-$C_3$-$C_8$-Cycloalkyl, oder eine Gruppe aus der Reihe

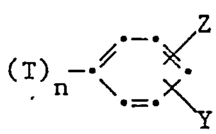 , $(T)_n$-Naphthyl(Y), $(T)_n$ -Si($C_1$-$C_8$-Alkyl)$_3$, (T)-$NR_2R_3$,

-N=$C(R_1)R_2$, (T)—P(=O)(OR$_3$)—($C_1$-$C_4$-Alkyl), (T)—P(=O)(OR$_3$)$_2$, oder $(T)_n$—W;

W einen 5- bis 7-gliedrigen gesättigten oder ungesättigten unsubstituierten oder durch Halogen, Trifluormethyl, Cyano oder $C_1$-$C_2$-Alkyl substituierten Heterocyclus mit 1 bis 3 Heteroatomen aus der Gruppe O, N und S sowie darüber hinaus einen Monosaccharid-Rest,

T unverzweigtes oder verzweigtes $C_1$-$C_8$-Alkylen, besonders-$CH_2$-, -$CH_2CH_2$-, -$CH_2$-$CH_2$-$CH_2$-, -CH($CH_3$)- oder -$CCH_3$($CH_3$)-, sowie $C_3$-$C_6$-Alkenylen oder $C_3$-$C_6$-Alkinylen,

T kann durch Sauerstoff oder CO unterbrochen sein und gegebenenfalls durch Halogen, Hydroxy, $C_1$-$C_5$-Alkoxy, $C_3$-$C_6$-Cycloalkyl, $C_3$-$C_5$-Alkoxycarbonyl, $C_1$-$C_5$-Alkyl, $C_1$-$C_2$-Haloalkyl, CN oder COOH substituiert sein, T kann auch Teil eines Cycloalkylrestes sein,

n Null oder 1,

$R_1$ und $R_2$ Wasserstoff, $C_1$-$C_6$-Alkyl, $C_2$-$C_8$-Alkoxyalkyl, Cyano oder durch Y substituiertes Phenyl, $C_1$-$C_3$-Alkanoyl, durch Halogen oder $C_1$-$C_3$-Alkoxy substituiertes $C_1$-$C_3$-Alkanoyl, $C_3$-$C_5$-Alkenoyl oder

durch Halogen oder $C_1$-$C_3$-Alkoxy substituiertes $C_3$-$C_5$-Alkenoyl,
$R_1$ und $R_2$ zusammen einen Heterocyclus W oder einen carbocyclischen Rest mit 3- bis 7 Ringkohlenstoffatomen,
Y und Z Wasserstoff, Halogen, $C_1$-$C_4$-Alkyl, $C_1$-$C_4$-Haloalkyl, $C_1$-$C_4$-Alkoxy, $C_1$-$C_4$-Haloalkoxy, Cyan, $NO_2$, $C_1$-$C_4$-Alkoxycarbonyl oder $C_1$-$C_4$-Alkylcarbonyl,
$R_3$ Wasserstoff oder $C_1$-$C_4$-Alkyl und
U Sauerstoff oder Schwefel
bedeuten.

3. Die 1,2,3-Benzthiadiazol-4-carbonsäure-Derivate der Formel I

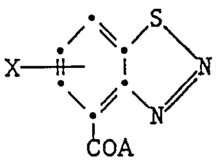

(I)

in welcher
X Wasserstoff, Halogen, $C_1$-$C_3$-Alkyl, $C_1$-$C_3$-Alkoxy oder $C_1$-$C_3$-Alkylthio,
A einen Rest -OR, -SR oder -ON = $C(R_1)R_2$,
R Wasserstoff, ein Alkalimetallkation, $C_1$-$C_8$-Alkyl, $C_3$-$C_4$-Alkenyl, $C_3$-$C_4$-Alkinyl, $C_1$-$C_3$-Cyclohexyl, Phenyl oder Benzyl wobei der Phenylkern durch $C_1$-$C_3$-Alkyl, $C_1$-$C_3$-Haloalkyl, $C_1$-$C_3$-Alkoxy, $C_1$-$C_3$-Alkylthio, Cyan, Halogen, Nitro oder Methoxycarbonyl substituiert sein kann,
$R_1$ und $R_2$ unabhängig voneinander je $C_1$-$C_6$-Alkyl, $C_2$-$C_8$-Alkoxyalkyl oder $R_1$ und $R_2$ zusammen eine 2-6 gliedrige Alkylen- oder Alkenylenkette, bedeuten.

4. 1,2,3-Benzthiadiazol-4-carbonsäure-Derivate gemäss Anspruch 1 der Formel Ia

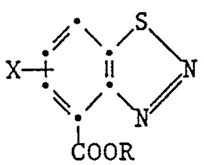

(Ia)

worin
X Wasserstoff, Halogen, $C_1$-$C_3$-Alkyl, $C_1$-$C_3$-Alkoxy oder $C_1$-$C_3$-Alkylthio,
R Wasserstoff, ein Alkalimetallkation, $C_1$-$C_8$-Alkyl, $C_3$-$C_4$-Alkenyl, $C_3$-$C_4$-Alkinyl, $C_3$-$C_6$-Cyclohexyl, Phenyl oder Benzyl wobei der Phenylkern durch $C_1$-$C_3$-Alkyl, $C_1$-$C_3$-Haloalkyl, $C_1$-$C_3$-Alkoxy, $C_1$-$C_3$-Alkylthio, Cyan oder Nitro substituiert sein kann.

5. 1,2,3-Benzthiadiazol-4-carbonsäure-Derivate gemäss Anspruch 4, ausgewählt aus
1,2,3-Benzthiadiazol-4-carbonsäuremethylester,
5-Chlor-1,2,3-benzthiadiazol-4-carbonsäuremethylester,
5-Chlor-1,2,3-benzthiadiazol-4-carbonsäure,
5-Chlor-1,2,3-benzthiadiazol-4-carbonsäureethylester,
5-Chlor-1,2,3-benzthiadiazol-4-carbonsäurebenzylester,
5-Methoxy-1,2,3-benzthiadiazol-4-carbonsäure-methylester,
5-Methylthio-1,2,3-benzthiadiazol-4-carbonsäure-methylester,
5-Chlor-1,2,3-benzthiadiazol-4-carbonsäure-n-butylester,
5-Chlor-1,2,3-benzthiadiazol-4-carbonsäure-phenylester,
5-Chlor-1,2,3-benzthiadiazol-4-carbonsäure-(2-chlorphenyl)-ester,
5-Chlor-1,2,3-benzthiadiazol-4-carbonsäure-propinylester,
5-Chlor-1,2,3-benzthiadiazol-4-carbonsäure-(trimethylsilylethyl)-ester,
5-Chlor-1,2,3-benzthiadiazol-4-carbonsäure-(dimethoxyphosphonylethyl)-ester und dem Kaliumsalz der 5-Chlor-4-(1,3-dioxan-2-yl)-1,2,3-benzthiazol-4-carbonsäure.

6. 1,2,3-Benzthiadiazol-4-carbonsäurethiolester gemäss Anspruch 1 der Formel Ib

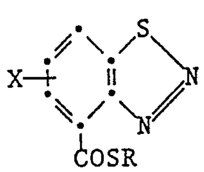

(Ib)

worin
X Wasserstoff, Halogen, $C_1$-$C_3$-Alkyl, $C_1$-$C_3$-Alkoxy oder $C_1$-$C_3$-Alkylthio,
R Wasserstoff, ein Alkalimetallkation, $C_1$-$C_8$-Alkyl, $C_3$-$C_4$-Alkenyl, $C_3$-$C_4$-Alkinyl, $C_3$-$C_6$-Cycloalkyl, Phenyl oder Benzyl wobei der Phenylkern durch $C_1$-$C_3$-Alkyl, $C_1$-$C_3$-Haloalkyl, $C_1$-$C_3$-Alkoxy, $C_1$-$C_3$-Alkylthio, Cyan oder Hitro substituiert sein kann.

7. 1,2,3-Benzthiadiazol-4-carbonsäurethiolester gemäss Anspruch 6, ausgewählt aus
5-Chlor-1,2,3-benzthiadiazol-4-carbonsäure-methylthiolester,
5-Methylthio-1,2,3-benzthiadiazol-4-carbonsäuremethylthiolester, und
5-Chlor-1,2,3-benzthiadiazol-4-carbonsäure-(methoxycarboylmethylthiol)-ester.

8. 1,2,3-Benzthiadiazol-4-carbonsäure-Derivate gemäss Anspruch 1 der Formel Ic

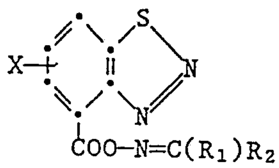

(Ic)

worin
X Wasserstoff, Halogen, $C_1$-$C_3$-Alkyl, $C_1$-$C_3$-Alkoxy oder $C_1$-$C_3$-Alkylthio,
$R_1$ und $R_2$ unabhängig voneinander je $C_1$-$C_6$-Alkyl, $C_2$-$C_8$-Alkoxyalkyl oder
$R_1$ und $R_2$ zusammen eine 2-6 gliedrige Alkylen- oder Alkenylenkette, bedeuten.

9. 5-Chlor-1,2,3-benzthiadiazol-4-carbonsäure-acetoxim-ester gemäss Anspruch 8.

10.Verfahren zur Herstellung der 1,2,3-Benzthiadiazol-4-carbonsäure-Derivate gemäss Anspruch 1 der Formel I

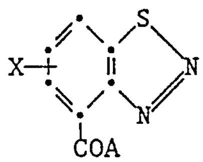

(I)

worin X und A die im Anspruch 1 gegebene Bedeutung haben, dadurch gekennzeichnet, dass man ein Säurehalid der Formel II

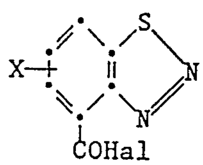

(II)

worin X die im Anspruch 1 gegebene Bedeutung hat und Hal für ein Halogenatom steht, in einem inerten organischen Lösungsmittel, in Gegenwart einer Base, mit einem Alkohol, Thiol oder Oxim der Formel III
H-A (III)
worin A eine der im Anspruch 1 gegebene Bedeutung hat, umsetzt.

11. Verfahren zur Herstellung der Verbindungen der Formel I gemäss Anspruch 1, dadurch gekennzeichnet, dass man einen 2-Amino-3-mercaptobenzoesäureester der Formel VII

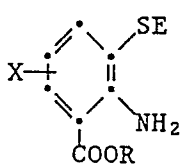

(VII)

worin X und R die im Anspruch 1 gegebene Bedeutung haben und E eine leicht abspaltbare Gruppe, ausgewählt aus Wasserstoff, Methyl, Ethyl, Isopropyl, Dodecyl oder Benzyl darstellt, in saurem Medium mit einem Alkalimetallnitrit bei -20° bis 30°C diazotiert, den erhaltenen 1,2,3-Benzthiadiazol-4-carbonsäureester der Formel Ic

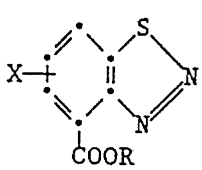

(Ic)

worin X und R die im Anspruch 1 gegebene Bedeutung haben, entweder als Endprodukt isoliert oder man verseift den Ester in wassriger Lösung mittels einer anorganischen Base zur 1,2,3-Benzthiadiazol-4-carbonsäure der Formel If

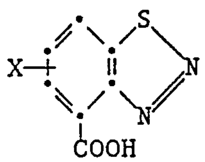

(If)

worin X die im Anspruch 1 gegebene Bedeutung hat, behandelt die Säure in einem inerten organischen Lösungsmittel mit einem Halogenisierungsmittel so, dass das Säurehalid der Formel II entsteht,

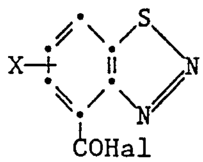

(II)

worin X die im Anspruch 1 gegebene Bedeutung hat und Hal für ein Halogenatom steht, und dieses in einem inerten organischen Lösungsmittel in Gegenwart einer Base, mit einem Alkohol, Thiol oder Oxim der Formel III

H-A (III),

worin A eine der im Anspruch 1 gegebene Bedeutung hat, umsetzt.

12. Pflanzenwuchsregulierendes Mittel, dadurch gekennzeichnet, dass es neben inerten Zutaten als Wirkstoffkomponente ein 1,2,3-Benzthiadiazol-4-carbonsäure-Derivat der Formel I enthält

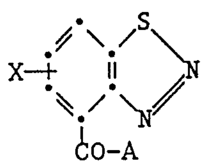

(I)

worin X und A die im Anspruch 1 gegebene Bedeutung haben.

13. Verfahren zur Hemmung des Pflanzenwuchses, dadurch gekennzeichnet, dass man Pflanzen, Pflanzenteile oder Samen mit einer wirksamen Menge eines 1,2,3-Benzthiadiazol-4-carbonsäure-Derivates gemäss Anspruch 1 oder einem Mittel welches ein solches Derivat enthält, behandelt.

14. Verfahren gemäss Anspruch 13 zur Hemmung des Pflanzenwuchses in Zuckerrohr-, Getreide-, Mais-, Soja-, Obst-, Reis- oder Baumwollkulturen oder bei Bodenbedecker-Leguminosen.

15. Verfahren gemäss Anspruch 13 zur Wuchshemmung im Sinne der Erhöhung der Standfestigkeit und des Ertrages in Kulturen von Soja.

16. 1,2,3-Benzthiadiazol-4-carbonsäurehalid der Formel II

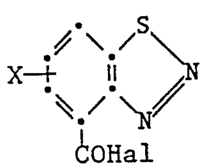

(II)

worin X die im Anspruch 1 gegebene Bedeutung hat und Hal ein Halogenatom darstellt, als Zwischenprodukt.

17. 2-Amino-3-mercapto-benzoesäure-Derivate der Formel VII

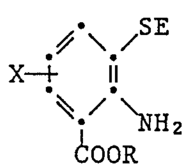

(VII)

worin X und R die im Anspruch 1 gegebene Bedeutung haben und E Wasserstoff, Methyl, Ethyl, Isopropyl, Dodecyl oder Benzyl bedeutet, als Zwischenprodukt.

18. Verfahren zur Herstellung der 2-Amino-3-mercapto-benzoesäure-Derivate der Formel VII gemäss Anspruch 17, dadurch gekennzeichnet, dass man ein 3-Halo-2-nitro-benzoesäure-Derivat der Formel V

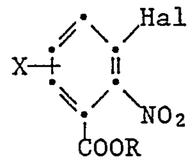

(V)

worin X und R die im Anspruch 1 gegebene Bedeutung hat und Hal für ein Halogenatom steht, in einem inerten organischen Lösungsmittel in Gegenwart der äquimolaren Menge einer Base mit einem Mercaptan der Formel IX

HS-E (IX)

umsetzt, worin E Wasserstoff, Methyl, Ethyl, Isopropyl, Dodecyl oder den Benzyl bedeutet und das entstandene 3-Mercapto-2-nitrobenzoesäure-Derivat der Formel VI

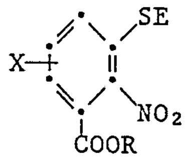

(VI)

worin X und R die im Anspruch 1 gegebene Bedeutung haben, und E Methyl, Ethyl, Isopropyl, Dodecyl oder Benzyl bedeutet, mit einem Reduktionsmittel zum 2-Amino-3-mercapto-benzoesäure-Derivat der Formel VII reduziert.

19. 3-Mercapto-2-nitrobenzoesäure-Derivat der Formel VI

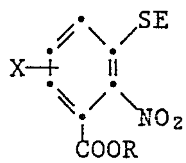

(VI)

worin X und R die im Anspruch 1 gegebene Bedeutung haben, und E Wasserstoff, Methyl, Ethyl, Isopropyl, Dodecyl oder Benzyl bedeutet, als Zwischenprodukt.